(19) Europäisches Patentamt
European Patent Office
Office européen des brevets

(11) **EP 4 458 316 A1**

(12) **EUROPEAN PATENT APPLICATION**
published in accordance with Art. 153(4) EPC

(43) Date of publication:
**06.11.2024 Bulletin 2024/45**

(21) Application number: **22916506.3**

(22) Date of filing: **06.12.2022**

(51) International Patent Classification (IPC):
*A61C 8/00* (2006.01)    *A01N 59/16* (2006.01)
*A61L 27/06* (2006.01)    *A61L 27/30* (2006.01)

(52) Cooperative Patent Classification (CPC):
**A01N 59/16; A61C 8/00; A61L 27/06; A61L 27/30**

(86) International application number:
**PCT/KR2022/019746**

(87) International publication number:
**WO 2023/128360 (06.07.2023 Gazette 2023/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.12.2021 KR 20210191745**

(71) Applicant: **Osstemimplant Co., Ltd.
Gangseo-gu
Seoul 07789 (KR)**

(72) Inventors:
• **HA, Kyungwon
Seoul 07789 (KR)**
• **HAN, Jaewon
Seoul 07789 (KR)**

(74) Representative: **Klunker IP
Patentanwälte PartG mbB
Destouchesstraße 68
80796 München (DE)**

(54) **DENTAL IMPLANT WITH EXCELLENT ANTIBACTERIAL PROPERTIES AND ANTIBACTERIAL COATING METHOD**

(57)    Disclosed is a dental implant including: a titanium base; a silver-titanium oxide matrix applied on at least a portion of the surface of the base, wherein the silver-titanium oxide matrix has a binding energy peak $P_1$ at 570 to 580 eV and a binding energy peak $P_2$ at 360 to 375 eV, measured by XPS, and the intensity $I_1$ of $P_1$ and the intensity $I_2$ of $P_2$ satisfy the following equation:

$$2^*I_1 \leq I_2 \leq 5^*I_1.$$

FIG. 10

**Description**

CROSS-REFERENCE TO RELATED APPLICATION

This application claims priority to and the benefit of Korean Patent Application No. 10-2021-0191745, filed on Dec 29, 2021, the disclosure of which is incorporated herein by reference in its entirety.

BACKGROUND

**1. Field of the Invention**

The present specification relates to a dental implant with excellent antibacterial properties and an antibacterial coating method.

**2. Discussion of Related Art**

Dental implants are artificial structures, which can permanently replace missing teeth, and are widely used to restore the chewing function in partially or completely edentulous areas. Therefore, dental implants not only have to function just like real teeth, but also need to be manufactured to properly distribute the load applied to the teeth so that they can be used for a long time.

The chances of successful dental implants are determined by implant stability, that is, the fixation force, depending on the patient's bone mass and bone quality. The stability of an implant is expressed as the sum of primary stability, which occurs when the implant comes in contact with the surrounding bone, and secondary stability, which occurs due to the formation of new bone tissue and osseointegration after implant placement. In particular, increasing the early stability after implant placement is crucial to increasing mid- to long-term stability and, ultimately, the success rate of implants.

The main cause of early implant failure is loss of bone surrounding the dental implant or decreased fixation force due to various reasons such as product defects, surgical factors, and patient-related factors. In particular, when the bone surrounding the implant is lost, the fixation force is inevitably decreased. As a result of analyzing early failure cases due to bone loss surrounding the implant, it was found that about 90% was caused by periodontitis or bacterial infection, and anaerobic bacteria were detected in about 60% of the products collected from customers.

In order to solve the above problems, a method was proposed to build a system that can actively defend against contaminated saliva and bacteria by imparting antibacterial properties to the implant surface. In particular, to reduce the early failure rate of implants, antibacterial properties need to be maintained even during the initial period after implant placement. Therefore, there is a need for research and development of an antibacterial coating method, which can maintain antibacterial properties during the initial period after implant placement by imparting antibacterial properties to the implant surface, and a dental implant accordingly.

SUMMARY OF THE INVENTION

The present specification is directed to providing a dental implant with excellent antibacterial properties and an antibacterial coating method.

According to an aspect of the present specification, there is provided a dental implant including: a titanium base; and a silver-titanium oxide matrix applied on at least a portion of the surface of the base, wherein the silver-titanium oxide matrix has a binding energy peak $P_1$ at 570 to 580 eV and a binding energy peak $P_2$ at 360 to 375 eV, measured by XPS, and the intensity $I_1$ of $P_1$ and the intensity $I_2$ of $P_2$ satisfy the following equation.

[Equation]

$$2*I_1 \leq I_2 \leq 5*I_1$$

In an embodiment, the dental implant may release 1 ppm/day or less of silver in an inorganic solvent at 37 °C for at least one week.

According to another aspect of the present specification, there is provided an antibacterial coating method for dental implants, which includes: (a) immersing a titanium base in a coating solution in which a silver precursor is dissolved and the Ag concentration is 10 to 300 mM; and (b) hydrothermally treating the coating solution by heating.

In an embodiment, the silver precursor may be one selected from the group consisting of silver nitrate ($AgNO_3$),

silver perchlorate ($AgClO_4$), silver tetrafluoroborate ($AgBF_4$), silver hexafluorophosphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), silver oxides ($AgO$, $Ag_2O$, $Ag_2O_2$, and $Ag_2O_3$) and mixtures of two or more thereof.

In an embodiment, before step (a), a pretreatment step of treating the titanium base through at least one method of sandblasting, etching, washing, and drying may be further included.

In an embodiment, step (b) may be performed under conditions of 150 to 300 °C and 1 to 5 hours.

BRIEF DESCRIPTION OF THE DRAWINGS

The above and other objects, features and advantages of the present specification will become more apparent to those of ordinary skill in the art by describing exemplary embodiments thereof in detail with reference to the accompanying drawings, in which:

FIG 1 is an antibacterial performance evaluation graph for selecting the antibacterial ion in the present specification;
FIG 2 is a set of removal torque graphs for selecting the antibacterial coating method of the present specification;
FIG 3 is an antibacterial performance evaluation graph according to hydrothermal treatment conditions;
FIG 4 shows FE-SEM analysis images of the example and comparative examples of the present specification;
FIG 5 shows a SEM image of the example of the present specification and results of analyzing the elemental distribution according to location;
FIG 6 shows EDS analysis images of the example and comparative examples of the present specification;
FIG 7 is an XPS analysis graph of the example and comparative examples of the present specification;
FIG 8 is a graph of released Ag amount in the example of the present specification;
FIG 9 is an antibacterial performance evaluation graph of the example of the present specification according to the immersion time; and
FIG 10 is an antibacterial performance evaluation graph of the example and comparative examples of the present specification according to washing conditions.

DETAILED DESCRIPTION OF EXEMPLARY EMBODIMENTS

Hereinafter, one aspect of the present specification will be described with reference to the attached drawings. However, the description in the present specification may be implemented in various forms, and therefore is not limited to the embodiments described herein. In the drawings, to clearly explain one aspect of the present specification, parts that are not related to the description are omitted, and similar parts are designated by similar reference numerals throughout the specification.

Throughout the specification, when a part is "connected" to another part, not only cases where they are "directly connected," but also cases where they are "indirectly connected" with another member in between are included. When a part "includes" a certain component, it means that other components can be further added rather than excluding other components, unless specifically stated to the contrary.

When a range of numerical values is described herein, unless otherwise specified, the value has the precision of significant figures given in accordance with the standard rules in chemistry for significant figures. For example, the number 10 includes a range of 5.0 to 14.9, and the number 10.0 includes a range of 9.50 to 10.49.

Hereinafter, an embodiment of the present specification will be described in detail with reference to the attached drawings.

Dental implant with excellent antibacterial properties

According to an aspect of the present specification, there is provided a dental implant including: a titanium base; and a silver-titanium oxide matrix applied on at least a portion of the surface of the base, wherein the silver-titanium oxide matrix has a binding energy peak $P_1$ at 570 to 580 eV and a binding energy peak $P_2$ at 360 to 375 eV, measured by XPS, and the intensity $I_1$ of $P_1$ and the intensity $I_2$ of $P_2$ satisfy the following equation.

[Equation]

$$2*I_1 \leq I_2 \leq 5*I_1$$

In order to increase the success rate of dental implants, it is necessary to form a stable bond with the user's surrounding bone during the initial period after implant placement. Among various causes of implant failure, early implant failure due to bacterial infection accounts for a large proportion.

The dental implant of the present specification may have antibacterial properties and include a titanium base; and a silver-titanium oxide matrix applied on at least a portion of the surface of the base. The silver-titanium oxide matrix may suppress the formation of a biofilm on the surface of the implant by blocking quorum sensing. Thus, the implant may minimize the chance of implant failure due to bacterial infection.

The phrase "quorum sensing" used herein refers to a bacterial density-dependent mechanism of regulating gene expression in which the collective metabolic activity of bacteria is regulated when bacteria accumulate and reach a specific concentration in proportion to cell density during bacterial growth. In other words, it refers to a phenomenon in which specific traits that are not observed in low-density cells are collectively induced and expressed when the concentration of bacteria reaches the so-called quorum for decision-making due to bacterial growth. In particular, pathogenic bacteria may cause infection by inducing virulence through quorum sensing.

The silver-titanium oxide matrix may kill bacteria by releasing silver ions, which are antibacterial ions. As a result, the attachment of bacteria to the implant surface may be suppressed. The silver ion may inactivate the protein by binding to the cysteine group of the bacterial membrane. The silver ion may kill bacteria by inducing the release of reactive oxygen species from the cells it binds to. Silver ions may effectively inhibit the formation of biofilms by killing bacteria through the mechanism described above and continuously maintain antibacterial properties without antibacterial resistance.

The silver-titanium oxide matrix may contain antibacterial components in various forms. As a result, it may have the level of antibacterial activity required at the initial period after implant placement and maintain the antibacterial effect for a long time.

The binding energy peak $P_1$ at 570 to 580 eV and $I_1$ may refer to the peak range and intensity value of Ag 2p, respectively. The binding energy peak $P_2$ at 360 to 375 eV and $I_2$ may refer to the peak range and intensity value of Ag 3d. Ag 2p may refer to silver metal, and Ag 3d may refer to oxidized silver ions. A silver-titanium oxide matrix in which $I_1$ and $I_2$, the intensities of these peaks, satisfy certain conditions may simultaneously contain the silver metal and silver ions to realize a sustained-release effect. When $I_1$ and $I_2$ satisfy certain conditions, the success rate of early placement of the dental implant may be effectively increased.

The conditions of $I_1$ and $I_2$, expressed in the above equation, may refer to the ratio between silver metal and silver ions measured in the XPS graph. $I_2$ may be 2 to 5 times greater than $I_1$. In an example, $I_2$ may be 2 times or more, 2.1 times or more, 2.2 times or more, 2.3 times or more, 2.4 times or more, 2.5 times or more, 2.6 times or more, 2.7 times or more, 2.8 times or more, 2.9 times or more, 3 times or more, 3.1 times or more, 3.2 times or more, 3.3 times or more, or 3.4 times or more than $I_1$. In another example, $I_2$ may be 5 times or less, 4.9 times or less, 4.8 times or less, 4.7 times or less, 4.6 times or less, 4.5 times or less, 4.4 times or less, 4.3 times or less, 4.2 times or less, 4.1 times or less, 4 times or less, 3.9 times or less, 3.8 times or less, 3.7 times or less, or 3.6 times or less than $I_1$.

Since a large amount of silver is released in a dental implant that satisfies the above conditions during the initial period, the dental implant may have antibacterial effects, such as killing bacteria and inhibiting adhesion, during the initial period after implant placement. In addition, the dental implant may continuously maintain antibacterial effects due to the sustained-release effect of gradually releasing silver.

For example, the dental implant may release 1 ppm/day or less of silver in an inorganic solvent at 37 °C for at least one week. The sustained-release effect of the dental implant may last for at least 1 week, 1 month, 2 months, 3 months, or longer. The sustained-release effect of the dental implant may mean achieving a continuous antibacterial effect by releasing at least one of silver ions and silver metal for a long time. The inorganic solvent may be water such as purified water or ion-exchanged water. The dental implant may release at least 1 ppb/day or more, 5 ppb/day or more, or 10 ppb/day or more of silver during the above period.

As a non-limiting example, the titanium base may be a material composed of pure titanium or an alloy of titanium and other metals in the periodic table. The titanium alloy may be, for example, one selected from the group consisting of combinations of titanium (Ti) with aluminum (Al), silicon (Si), vanadium (V), niobium (Nb), zirconium (Zr), molybdenum (Mo), chromium (Cr), tin (Sn), tantalum (Ta), palladium (Pd), and one or more thereof, but is not limited thereto.

Antibacterial coating method for dental implants

According to another aspect of the present specification, there is provided an antibacterial coating method for dental implants, which includes: (a) immersing a titanium base in a coating solution in which a silver precursor is dissolved and the Ag concentration is 10 to 300 mM; and (b) hydrothermally treating the coating solution by heating.

In step (a), a titanium base may be immersed in a coating solution in which a silver precursor is dissolved. The silver precursor may be one selected from the group consisting of silver nitrate ($AgNO_3$), silver perchlorate ($AgClO_4$), silver tetrafluoroborate ($AgBF_4$), silver hexafluorophosphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), silver oxides ($AgO$, $Ag_2O$, $Ag_2O_2$, and $Ag_2O_3$) and mixtures of two or more thereof, but is not limited thereto.

As an example, the Ag concentration of the coating solution may be 10 to 300 mM. For example, it may be 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM, 30 mM, 31 mM, 32 mM, 33 mM, 34 mM, 35 mM, 36 mM, 37 mM, 38 mM, 39 mM, 40 mM, 41 mM, 42 mM, 43 mM, 44 mM, 45 mM, 46 mM, 47 mM, 48 mM, 49 mM, 50 mM, 51 mM, 52 mM, 53 mM, 54 mM, 55 mM, 56 mM, 57 mM, 58 mM, 59 mM, 60 mM, 61 mM, 62 mM, 63 mM, 64 mM, 65 mM, 66 mM, 67 mM, 68 mM, 69 mM, 70 mM, 71 mM, 72 mM, 73 mM, 74 mM, 75 mM, 76 mM, 77 mM, 78 mM, 79 mM, 80 mM, 81 mM, 82 mM, 83 mM, 84 mM, 85 mM, 86 mM, 87 mM, 88 mM, 89 mM, 90 mM, 91 mM, 92 mM, 93 mM, 94 mM, 95 mM, 96 mM, 97 mM, 98 mM, 99 mM, 100 mM, 110 mM, 120 mM, 130 mM, 140 mM, 150 mM, 160 mM, 170 mM, 180 mM, 190 mM, 200 mM, 210 mM, 220 mM, 230 mM, 240 mM, 250 mM, 260 mM, 270 mM, 280 mM, 290 mM, 300 mM or a range between two of these values, but is not limited thereto. When the Ag concentration is less than the above range, antibacterial properties may be reduced due to an insufficient amount of silver, and when the Ag concentration exceeds the above range, it may be difficult to form a coating solution or the formation of a coating layer may be poor due to problems such as viscosity.

In an example, before step (a), a pretreatment step of treating the titanium base through at least one method of sandblasting, etching, washing, and drying may be further included, but is not limited thereto.

When surface roughness is imparted to the titanium base through sandblasting or etching, osseointegration may be improved, but adhesion to bacteria may also be increased. By washing or drying the titanium base, foreign substances on the surface of the base may be removed, and the subsequent antibacterial coating layer may be effectively formed.

In step (b), a silver-titanium oxide matrix may be formed on the surface of the titanium base by thermally treating the coating solution through heating.

In an example, step (b) may be performed at 150 to 300 °C and 1 to 5 hours. Step (b) may be performed, for example, at a temperature of 150 °C, 155 °C, 160 °C, 165 °C, 170 °C, 175 °C, 180 °C, 185 °C, 190 °C, 195 °C, 200 °C, 205 °C, 210 °C, 215 °C, 220 °C, 225 °C, 230 °C, 235 °C, 240 °C, 245 °C, 250 °C, 255 °C, 260 °C, 265 °C, 270 °C, 275 °C, 280 °C, 285 °C, 290 °C, 295 °C, 300 °C or a range between two of these values, but is not limited thereto. The temperature condition of step (b) may affect the silver coating amount of the silver-titanium oxide matrix and the resulting antibacterial performance. In addition, step (b) is performed, for example, for 1 hour, 1.5 hours, 2 hours, 2.5 hours, 3 hours, 3.5 hours, 4 hours, 4.5 hours, 5 hours, or a time range between two of these values, but is not limited thereto.

After step (b), the surface of the titanium base may be washed to remove unbound silver ions. However, such washing may be performed in a way that does not impair the antibacterial performance of the silver-titanium oxide matrix formed on the surface of the titanium base. For example, it may be performed by immersing the base in one selected from the group consisting of purified water, alcohol, and mixtures thereof and then stirring.

Hereinafter, the examples of the present specification will be described in more detail. However, the following experimental results describe only representative results among the above examples, and the scope and content of the present specification cannot be construed as being reduced or limited by the examples. Each effect of various implementations of the present specification that are not explicitly presented below will be described in detail in the corresponding section.

Experimental Example 1: Selection of antibacterial ion

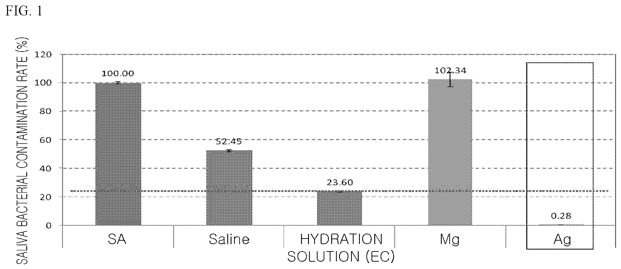

To select the antibacterial ion, the contamination rate of bacteria in saliva was evaluated. Saliva (SA) was prepared as a control group, and 100 ml of a saline solution (Saline), a hydration solution (EC), a magnesium ion salt, and a silver ion salt were prepared as experimental groups. 1 ml of a saliva bacterial suspension separated from saliva was added to each solution of the control and experimental groups and maintained at 37 °C under anaerobic conditions for 16 hours. After 16 hours, the saliva bacterial contamination rate was measured and the results thereof are shown in FIG 1. The saliva bacterial contamination rate of the control group, saliva, was set to 100%, and the saliva bacterial contamination rate of the experimental groups was measured.

Referring to FIG 1, the saline solution (Saline) and hydration solution (EC) had saliva bacterial contamination rates of 52.45% and 23.6%, respectively, compared to saliva. The magnesium ion salt, an antibacterial ionic salt, had the highest saliva bacterial contamination rate of 102.34%. On the other hand, the silver ion salt had a saliva bacterial contamination rate of 0.28%, which was a 99.7% reduction in bacterial contamination compared to saliva. Therefore, silver ions were selected as antibacterial ions.

Experimental Example 2: Selection of antibacterial coating method

To select the antibacterial coating method, animal experiments were performed. A titanium implant was prepared as a control group. As experimental groups, titanium implants treated with a solution containing a silver ion salt and hydrogen peroxide and titanium implants hydrothermally treated with a solution containing a silver ion salt were prepared.

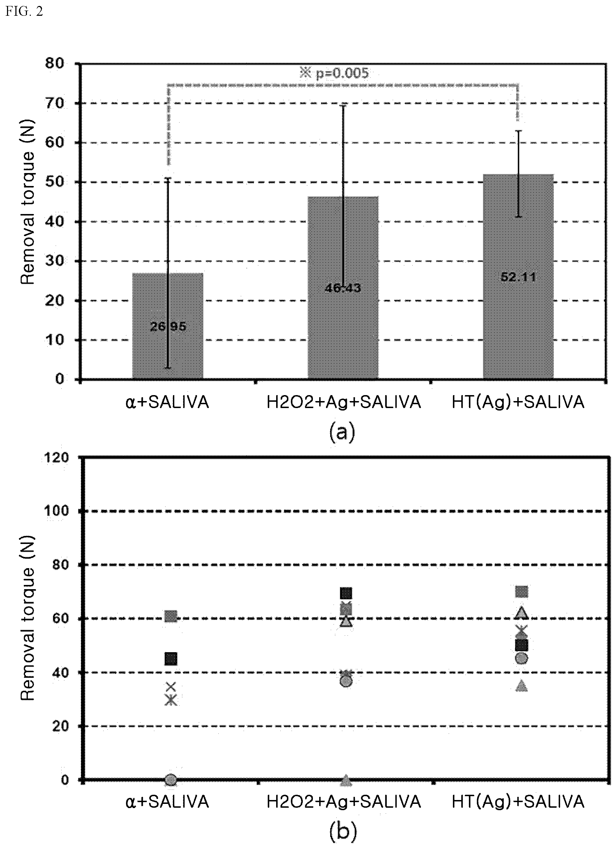

Eight implant samples were prepared for each group. The prepared implants were contaminated by immersing them in saliva for 24 hours before implant placement and were placed in the tibia of a rabbit and maintained for 21 days for bone formation, and then the removal torque was evaluated. The experimental results thereof are shown in FIG 2.

Referring to FIG 2, FIG 2A shows the average value of the removal torque for each of the eight samples. FIG 2B shows the removal torque for each of the eight samples. The untreated titanium implants were the lowest with an average removal torque value of 26.95N, and the number of implant failures was 2 out of 8. On the other hand, given that the silver ion-coated implants had values of 46.43N and 52.11N, it can be confirmed that saliva contamination is reduced due to silver ions. The implants coated with silver ions through hydrothermal treatment had the highest removal torque, and the number of implant failures was 0 out of 8. Therefore, it was confirmed that silver ions had excellent antibacterial properties against salivary bacteria, and hydrothermal treatment was selected as the coating method.

Experimental Example 3: Selection of hydrothermal treatment conditions 1

To select hydrothermal treatment conditions, antibacterial performance was evaluated according to temperature, time, and silver ion concentration.

For evaluation, nine titanium bases with a size of 3x3 cm$^2$ were prepared. For the hydrothermal treatment conditions for each titanium base, the temperature was set to 180 °C, 200 °C, and 250 °C, the time was set to 1 hour, 2 hours, and 3 hours, and the silver ion concentration was set to 30 mM, 60 mM, and 100 mM.

As salivary bacteria, Aggregatibacter actinomycetemcomitans ATCC 33384 and Porphyromonas gingivalis, which are causative bacteria of acute periodontitis or peri-implantitis, were used. The prepared salivary bacteria were each inoculated into a brain heart infusion (BHI) broth (Merck KGaA) and cultured for 16 to 24 hours under anaerobic conditions at 37 °C. The culture medium was diluted in BHI medium so that the absorbance at 600 nm was 0.1 and then diluted in BHI medium one more time so that the bacterial concentration was 1/10 to prepare two types of saliva bacterial suspensions.

The nine titanium bases were sterilized through exposure to UV light on a clean bench for 30 minutes and then placed into each well of a 24 well plate. 1 mL of the prepared saliva bacterial suspension was added to each well and cultured for 16 hours at 37 °C under anaerobic conditions. 1 mL of a 0.1 wt% crystal violet solution was added to each well of a new 24 well plate. The samples were transferred to each well of the well plate containing crystal violet and cultured at room temperature for 10 minutes to stain the bacteria purple.

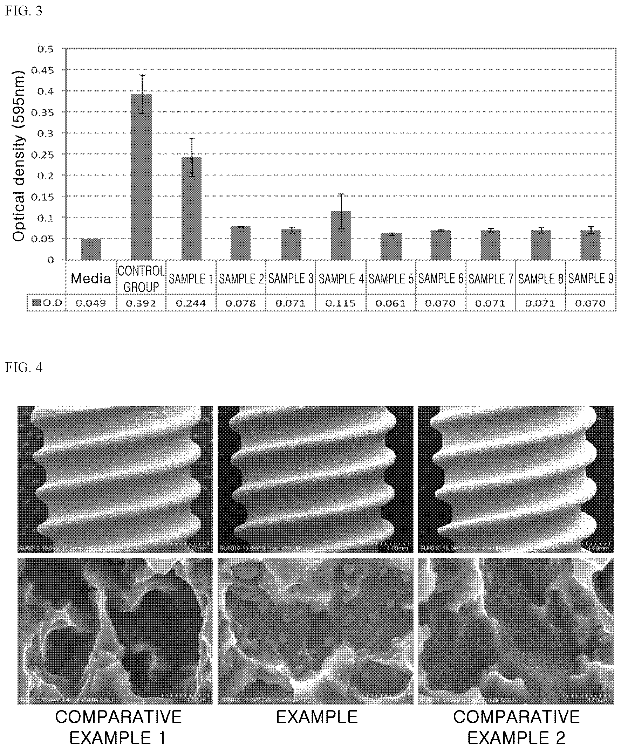

A new 24 well plate was prepared by adding 1 mL of a phosphate buffered saline (PBS) solution to each well, and then the stained samples were transferred to each well, and the above process was repeated one more time. The samples washed with the PBS solution were moved onto a dry tissue and the bottom of the samples was cleaned. 500 μL of a 30 wt% acetic acid solution was added to each well of a new 24 well plate, and then the samples were transferred to each well and cultured at room temperature for 20 minutes to dissolve the dye. After adding 200 μL of the dissolved solution to a 96 well plate, absorbance (595 nm) was measured using a microplate reader, and the results thereof are shown in FIG 3. For comparison with the samples, absorbance (O.D 595 nm) was measured for a medium uninoculated with saliva bacteria (Media) and a control group in which the untreated titanium base sample was treated with the saliva bacterial suspension as described above.

The hydrothermal treatment conditions for each of the nine samples are shown in Table 1 below.

[Table 1]

| Classification | Temperature (°C) | Time (h) | Ag ion concentration (mM) | Absorbance (595 nm) |
|---|---|---|---|---|
| Sample 1 | 180 | 1 | 30 | 0.244 |
| Sample 2 | 180 | 2 | 60 | 0.078 |
| Sample 3 | 180 | 3 | 100 | 0.071 |
| Sample 4 | 200 | 1 | 60 | 0.115 |
| Sample 5 | 200 | 2 | 100 | 0.061 |
| Sample 6 | 200 | 3 | 30 | 0.070 |
| Sample 7 | 250 | 1 | 100 | 0.071 |
| Sample 8 | 250 | 2 | 30 | 0.071 |
| Sample 9 | 250 | 3 | 60 | 0.070 |

Referring to Table 1 and FIG 3, all samples except Sample 1 and Sample 4 had an O.D (595 nm) value of 0.08 or less, and thus it was confirmed that the silver ion has an excellent salivary bacteria inhibition effect.

In order to derive the optimal hydrothermal treatment conditions, the O.D (595 nm) values were summed according to temperature, time, and silver ion concentration and are shown in Table 2.

[Table 2]

| Classification | Condition | Absorbance (595 nm) | Difference (Maximum - Minimum) |
|---|---|---|---|
| Temperature (°C) | 180 | 0.393 | 0.181 |
| | 200 | 0.246 | |
| | 250 | 0.212 | |
| Time (h) | 1 | 0.430 | 0.22 |
| | 2 | 0.210 | |
| | 3 | 0.211 | |
| Ag ion concentration (mM) | 30 | 0.385 | 0.182 |
| | 60 | 0.263 | |
| | 100 | 0.203 | |

Referring to Table 2, the O.D (595 nm) values according to the temperature in hydrothermal treatment were 0.246 and 0.212 at 200 °C and 250 °C, respectively, confirming that antibacterial properties were improved compared to the case of performing hydrothermal treatment at 180 °C. The saliva bacterial contamination rate according to time was low under the conditions of 2 hours and 3 hours, and the saliva bacterial contamination rate according to the silver ion concentration was low at the concentrations of 60 mM and 100 mM. Therefore, the optimal hydrothermal treatment conditions for improving antibacterial properties were determined to be a temperature of 200 to 250 °C, a time of 2 to 3 hours, and a silver ion concentration of 60 to 100 mM.

Experimental Example 4: Selection of hydrothermal treatment conditions 2

To select hydrothermal treatment conditions, the saliva bacterial contamination inhibition rate, initial eluted titanium (Ti) ion amount, and released silver (Ag) ion amount were evaluated, according to $H_2O_2$ concentration (chemical treatment), temperature, and time during hydrothermal treatment.

For evaluation, nine titanium bases with the size of 3x3 cm$^2$ were prepared. For the hydrothermal treatment conditions for each titanium base, the $H_2O_2$ concentration was set to 0 wt% (no addition), 1 wt%, and 2 wt%, the temperature was set to 180 °C, 200 °C, and 250 °C, the time was set to 1 hour, 2 hours, and 3 hours, and the silver ion concentration was fixed at 100 mM.

The evaluation for the saliva bacterial contamination inhibition rate was conducted in the same manner as in the antibacterial performance evaluation described above in Experimental Example 3, and the saliva bacterial contamination inhibition rate was derived based on the O.D (595 nm) value of the untreated titanium base sample contaminated with saliva bacteria.

Saliva bacterial contamination inhibition rate (%) = (Measured absorbance of antibacterial coated sample according to hydrothermal treatment conditions)/ (Measured absorbance of untreated sample)

In order to derive the optimal hydrothermal treatment conditions, the sum of the saliva bacterial contamination inhibition rate, the initial eluted Ti amount, and the released Ag amount according to $H_2O_2$ concentration, temperature, and time are shown in Table 3.

[Table 3]

| Classification | Condition | Saliva bacterial contamination inhibition rate (%) | | Released Ag amount (ppb) | | Initial eluted Ti amount (ppb) | |
|---|---|---|---|---|---|---|---|
| | | Sum | Difference (Maximum - Minimum) | Sum | Difference (Maximum - Minimum) | Sum | Difference (Maximum - Minimum) |
| $H_2O_2$ concentration | 0% | 2.89 | | 1517.42 | | 10.32 | |
| | 1% | 2.96 | 0.08 | 1774.65 | 772.7 | 178.16 | 668.03 |
| | 2% | 2.97 | | 2290.12 | | 678.35 | |
| Temperature | 150°C | 2.93 | | 947.67 | | 689.47 | |
| | 200°C | 2.93 | 0.04 | 3271.52 | 2323.8 | 31.21 | 658.26 |
| | 250°C | 2.97 | | 1363 | | 146.15 | |
| Time | 1h | 2.92 | | 1675.6 | | 179.19 | |
| | 2h | 2.93 | 0.06 | 2138.9 | 463.3 | 541.11 | 394.58 |
| | 3h | 2.98 | | 1767.69 | | 146.53 | |

Referring to Table 3, it was confirmed that during hydrothermal treatment, the saliva bacterial contamination inhibition rate according to the $H_2O_2$ concentration had a slight difference, with the difference between the maximum and minimum values being 0.08, but the initial eluted Ti amount significantly increased as the concentration increased. Thus, it was decided not to use $H_2O_2$ during hydrothermal treatment. In addition, in terms of the temperature condition, there was a significant result in that a large amount of Ag ions were released at 200 °C or higher while a small amount of Ti ions were initially eluted. In terms of the saliva bacterial contamination inhibition rate according to the time condition, the highest inhibition rate was at 3 hours, the released Ag amount was highest at 2 hours, and the initial eluted Ti amount was lowest at 3 hours.

An Example and Comparative Examples were set according to the selection of antibacterial ions and hydrothermal treatment conditions through Experimental Examples 1 to 4 described above.

Example

A Cp-Ti (Titanium Grade 4) sample in the form of a disk (cylinder) with a diameter of 6 mm, a thickness of 2 mm, and a length of 10 mm was prepared. The sample was immersed in a silver nitrate solution with an Ag concentration of 100 mM and subjected to hydrothermal treatment at a temperature of 200 °C for 3 hours. After hydrothermal treatment, the sample was immersed in deionized water for 5 minutes and washed to complete the antibacterial coating on the sample.

Comparative Example 1

The same manner as in the Example was performed, except that the silver precursor solution was replaced with deionized water and hydrothermal treatment was performed.

Comparative Example 2

The same manner as in the Example was performed, except that after hydrothermal treatment, it was immersed in deionized water and subjected to ultrasonic cleaning for 5 minutes.

Experimental Example 5: FE-SEM and EDS analysis

FE-SEM and EDS analyses were performed for surface analysis of the Example and Comparative Examples, and the results thereof are shown in FIGS. 4 to 6.

FIG 4 is a set of FE-SEM analysis images of the Example and Comparative Examples of the present specification. Referring to FIG 4, it can be seen that silver particles were formed on the surface of the Example. On the other hand, it can be seen that coating particles were not formed on the surface of Comparative Example 1 and the coating of silver particles was reduced in Comparative Example 2 compared to the Example.

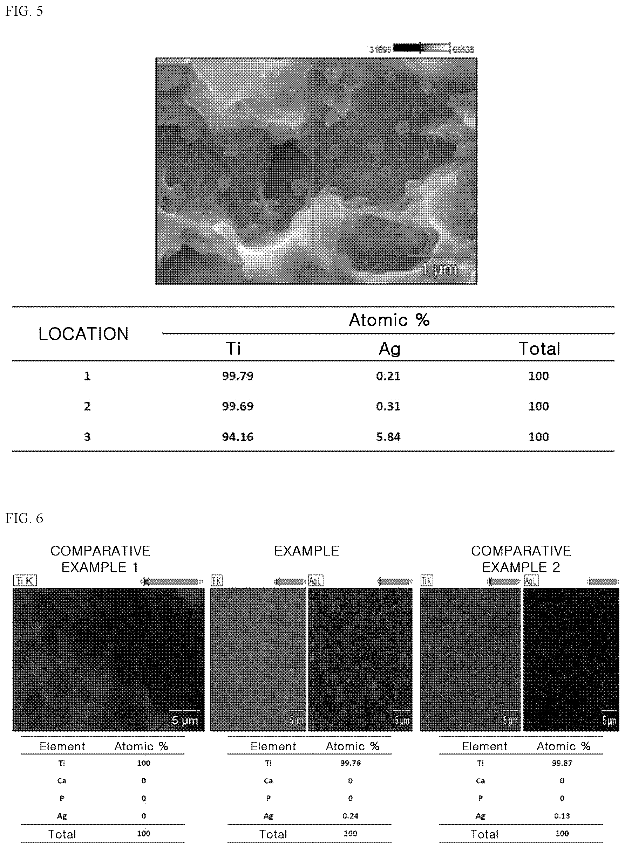

FIG 5 shows a SEM image of the Example and an analysis of the elemental distribution of titanium and silver according to location. Referring to FIG 5, Location 1 had 99.79 at% titanium and 0.21 at% silver, Location 2 had 99.69 at% titanium and 0.31 at% silver, and Location 3 had 94.16 at% titanium and 5.84 at% silver. It can be seen that bulk silver particles were formed at Location 3 and the at% of silver increased.

FIG 6 is a set of EDS analysis images of the Example and Comparative Examples of the present specification. Referring to FIG 6, it can be seen that silver is evenly distributed on the titanium base surface of the Example. In Comparative Example 1, the antibacterial coating layer was not formed and only the titanium element was distributed. In Comparative Example 2, silver was evenly distributed on the surface of the titanium base, but a smaller amount of silver was distributed compared to the Example.

Experimental Example 6: XPS analysis

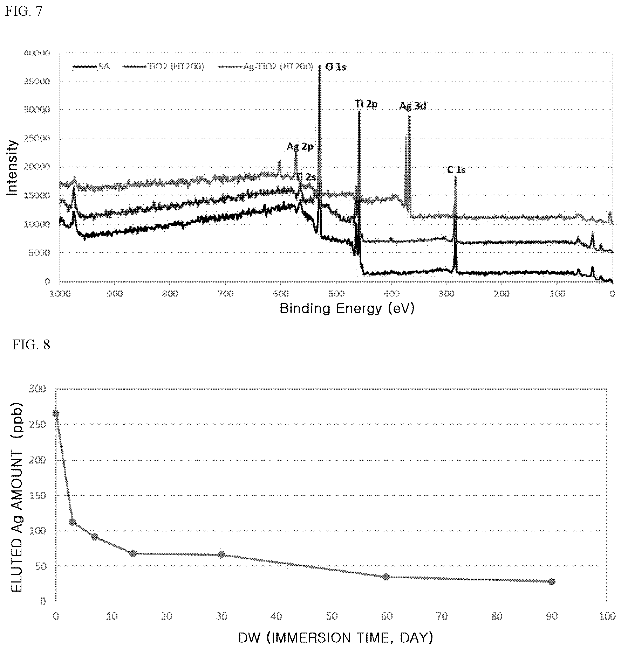

XPS analysis was performed to analyze the atomic state of the Example and Comparative Examples, and the results are shown in FIG 7. XPS analysis was also performed on an untreated Cp-Ti (Titanium Grade 4) sample as a control group.

Referring to FIG 7, it can be seen that Ag metal (Ag 2p) and oxidized Ag ion (Ag 3d) peaks were simultaneously detected in the analysis graph of the Example. Therefore, it can be confirmed that the titanium base surface was effectively coated with silver particles in the Example. On the other hand, in Comparative Examples, Ag peaks were not detected, and only Ti peaks (Ti 2s, Ti 2p) were detected. It can be seen that the control group had a peak pattern almost similar to those of Comparative Examples, but the weak oxidized Ti ion peak appeared because surface treatment was not performed.

Comparing the height of the Ag metal peak and the oxidized Ag ion peak, it can be seen that the peak of Ag 3d at 360 to 375 eV is about 3.5 times higher than the peak of Ag 2p at 570 to 580 eV

Experimental Example 7: Released Ag amount evaluation

To evaluate the amount of released Ag of the Example according to immersion time, the sample of the Example was immersed in deionized water (DW), and the amount of Ag released over 90 days was measured. The released Ag amount was measured using an ICP-MS analyzer, and the results thereof are shown in FIG 8.

Referring to FIG 8, the released Ag amount was 250 ppb or more immediately after immersion, and the released Ag amount was 100 ppb or more after 3 days of immersion. It can be seen that after 3 days of immersion, the released Ag amount gradually decreased, and after 30 days, 50 ppb or more of Ag ions were slowly released. After 60 days of immersion, 30 ppb or more of Ag ions were released. Therefore, when the Example of the present specification is applied to dental implants, silver ions, which are antibacterial ions, may be released slowly during the initial period after implant placement (about 3 months), and thus, the early implant failure rate is expected to be significantly reduced.

Experimental Example 8: Antibacterial performance evaluation according to Ag ion release period

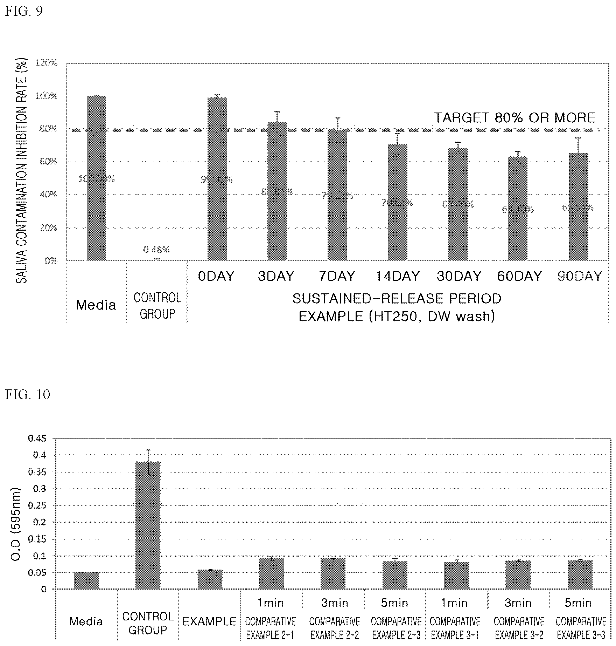

The antibacterial performance of the Example was evaluated according to the Ag ion release period. For comparison with the Example, the saliva bacterial contamination inhibition rate was measured in a medium uninoculated with saliva bacteria (Media) and a medium containing only a saliva bacterial suspension (the control group), and the results are shown in FIG 9.

Referring to FIG 9, according to the Ag ion release period of the Example, the saliva bacterial contamination inhibition rate was more than 80% for 3 days. For 90 days, the saliva bacterial contamination inhibition rate gradually decreased but was over 60%. It can be confirmed that Ag ions were slowly released within 90 days.

Experimental Example 9: Antibacterial performance evaluation according to washing conditions

Antibacterial performance was evaluated according to the Ag washing conditions of Example and Comparative Example. For evaluation, the Example and Comparative Example 2, which was ultrasonically cleaned with deionized water after hydrothermal treatment, were selected. For Comparative Example 2, the ultrasonic cleaning time was subdivided into 1 minute, 3 minutes, and 5 minutes and set as Comparative Example 2-1, Comparative Example 2-2, and Comparative Example 2-3, respectively. The washing solution was replaced with ethanol and set as Comparative Example 3, and the ultrasonic cleaning time was subdivided into 1 minute, 3 minutes, and 5 minutes, and set as Comparative Example 3-1, Comparative Example 3-2, and Comparative Example 3-3. The antibacterial performance evaluation was conducted in the same manner as in Experimental Example 3 to measure the absorbance, the O.D (595 nm) value, and the results thereof are shown in FIG 10.

Referring to FIG 10, the Example, in which the sample was immersed in deionized water and cleaned, had the best antibacterial properties. On the other hand, Comparative Examples 2 (2-1, 2-2, 2-3) and 3 (3-1, 3-2, 3-3) in which ultrasonic cleaning was performed, had a slight decrease in antibacterial performance. It is predicted that some of the bulk ion particles were removed by ultrasonic cleaning, affecting antibacterial performance. It can be seen that the antibacterial performance according to washing solutions had almost no effect.

According to an aspect, a dental implant with excellent antibacterial properties and an antibacterial coating method can improve the antibacterial properties of the implant due to a silver-titanium oxide matrix formed on the implant surface. In the silver-titanium oxide matrix, silver ions are slowly released during the initial period after implant placement, thereby imparting antibacterial properties to the implant and maintaining them. Thus, the early failure rate of the dental implant can be reduced, and ultimately, the success rate of the implant can be increased.

The effects of an aspect of the present specification are not limited to the effects described above and should be understood to include all effects that can be inferred from the configuration described in the detailed description or claims of the present specification.

The above description of the present specification is for illustrative purposes, and those skilled in the art to which an aspect of the present specification pertains can understand that they can easily transform it into another specific form without changing the technical idea or essential features described in the present specification. Therefore, the embodiments described above should be understood in all respects as illustrative and not restrictive. For example, each component described as unitary may be implemented in a distributed manner, and likewise, each component described as distributed may also be implemented in a combined form.

The scope of the present specification is indicated by the claims described below, and all changes or modified forms derived from the meaning and scope of the claims and their equivalent concepts should be construed as being included in the scope of the present specification.

**Claims**

1. A dental implant comprising:

    a titanium base; and
    a silver-titanium oxide matrix applied on at least a portion of the surface of the base,
    wherein the silver-titanium oxide matrix has a binding energy peak $P_1$ at 570 to 580 eV and a binding energy peak $P_2$ at 360 to 375 eV, measured by XPS, and
    the intensity $I_1$ of $P_1$ and the intensity $I_2$ of $P_2$ satisfy the following equation:

    [Equation]

    $$2*I_1 \leq I_2 \leq 5*I_1.$$

2. The dental implant of claim 1, wherein 1 ppm/day or less of silver is released in an inorganic solvent at 37 °C for at least one week.

3. An antibacterial coating method for dental implants, comprising:

    (a) immersing a titanium base in a coating solution in which a silver precursor is dissolved and the Ag concentration is 10 to 300 mM; and
    (b) hydrothermally treating the coating solution by heating.

4. The method of claim 3, wherein the silver precursor is one selected from the group consisting of silver nitrate ($AgNO_3$), silver perchlorate ($AgClO_4$), silver tetrafluoroborate ($AgBF_4$), silver hexafluorophosphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), silver oxides ($AgO$, $Ag_2O$, $Ag_2O_2$, and $Ag_2O_3$) and mixtures of two or more thereof.

5. The method of claim 3, further comprising a pretreatment step of treating the titanium base through at least one method of sandblasting, etching, washing, and drying, before step (a).

6. The method of claim 3, wherein step (b) is performed under conditions of 150 to 300 °C and 1 to 5 hours.

FIG. 1

FIG. 2

(a)

(b)

FIG. 3

| | Media | CONTROL GROUP | SAMPLE 1 | SAMPLE 2 | SAMPLE 3 | SAMPLE 4 | SAMPLE 5 | SAMPLE 6 | SAMPLE 7 | SAMPLE 8 | SAMPLE 9 |
|---|---|---|---|---|---|---|---|---|---|---|---|
| O.D | 0.049 | 0.392 | 0.244 | 0.078 | 0.071 | 0.115 | 0.061 | 0.070 | 0.071 | 0.071 | 0.070 |

FIG. 4

COMPARATIVE EXAMPLE 1     EXAMPLE     COMPARATIVE EXAMPLE 2

FIG. 5

| LOCATION | Atomic % | | |
|---|---|---|---|
| | Ti | Ag | Total |
| 1 | 99.79 | 0.21 | 100 |
| 2 | 99.69 | 0.31 | 100 |
| 3 | 94.16 | 5.84 | 100 |

FIG. 6

COMPARATIVE EXAMPLE 1

| Element | Atomic % |
|---|---|
| Ti | 100 |
| Ca | 0 |
| P | 0 |
| Ag | 0 |
| Total | 100 |

EXAMPLE

| Element | Atomic % |
|---|---|
| Ti | 99.76 |
| Ca | 0 |
| P | 0 |
| Ag | 0.24 |
| Total | 100 |

COMPARATIVE EXAMPLE 2

| Element | Atomic % |
|---|---|
| Ti | 99.87 |
| Ca | 0 |
| P | 0 |
| Ag | 0.13 |
| Total | 100 |

FIG. 7

FIG. 8

FIG. 9

FIG. 10

## INTERNATIONAL SEARCH REPORT

| International application No. |
| --- |
| **PCT/KR2022/019746** |

| A. | CLASSIFICATION OF SUBJECT MATTER |
| --- | --- |

**A61C 8/00**(2006.01)i; **A01N 59/16**(2006.01)i; **A61L 27/06**(2006.01)i; **A61L 27/30**(2006.01)i

According to International Patent Classification (IPC) or to both national classification and IPC

| B. | FIELDS SEARCHED |
| --- | --- |

Minimum documentation searched (classification system followed by classification symbols)

A61C 8/00(2006.01); A01N 59/16(2006.01); A61F 2/28(2006.01); C01B 11/22(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 치과용 임플란트 코팅(dental implant coating), 항균(antimicrobial), 티타늄 (titanium), 은이온(silver ion), 침지(soak), 수열처리(hydrothermal treatment)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-2013-0059421 A (INNOVOTECH, INC.) 05 June 2013 (2013-06-05)<br>See paragraphs [0034]-[0041], [0052], [0065], [0090], [0100]-[0102], [0129]-[0135] and [0173]-[0184]. | 3-6 |
| A | | 1-2 |
| X | WANG, Jiaxing et al. Silver-nanoparticles-modified biomaterial surface resistant to staphylococcus: New insight into the antimicrobial action of silver. Scientific Reports. 2016, vol. 6, Article No. 32699, pp. 1-16.<br>See abstract; pages 2 and 12; and figure 1. | 3-6 |
| A | US 2011-0046747 A1 (YEUNG, Kelvin Wai Kwok et al.) 24 February 2011 (2011-02-24)<br>See entire document. | 1-6 |
| A | MOKABBER, T. et al. Antimicrobial electrodeposited silver-containing calcium phosphate coatings. ACS Applied Materials & Interfaces. 2020, vol. 12, pp. 5531-5541.<br>See entire document. | 1-6 |

| ✓ Further documents are listed in the continuation of Box C. | ✓ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 March 2023** | **17 March 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/019746**

**C.    DOCUMENTS CONSIDERED TO BE RELEVANT**

| Category* | Citation of document, with indication, where appropriate, of the relevant passages | Relevant to claim No. |
|---|---|---|
| A | ZHENG, Tian-Xia et al. Classification and research progress of implant surface antimicrobial techniques. Journal of Dental Sciences. 2022 (online publication date: 20 September 2021), vol. 17, pp. 1-7. See entire document. | 1-6 |

Form PCT/ISA/210 (second sheet) (July 2022)

INTERNATIONAL SEARCH REPORT
Information on patent family members

International application No.

**PCT/KR2022/019746**

| Patent document cited in search report | | | Publication date (day/month/year) | Patent family member(s) | | | Publication date (day/month/year) |
|---|---|---|---|---|---|---|---|
| KR | 10-2013-0059421 | A | 05 June 2013 | CA | 2808930 | A1 | 23 February 2012 |
| | | | | EP | 2605659 | A1 | 26 June 2013 |
| | | | | EP | 2605659 | B1 | 08 July 2020 |
| | | | | EP | 3804521 | A1 | 14 April 2021 |
| | | | | US | 2012-0058169 | A1 | 08 March 2012 |
| | | | | US | 2014-0328890 | A1 | 06 November 2014 |
| | | | | US | 2018-0168165 | A1 | 21 June 2018 |
| | | | | WO | 2012-021979 | A1 | 23 February 2012 |
| US | 2011-0046747 | A1 | 24 February 2011 | CN | 102341132 | A | 01 February 2012 |
| | | | | CN | 102341132 | B | 13 November 2013 |
| | | | | EP | 2398517 | A2 | 28 December 2011 |
| | | | | EP | 2398517 | B1 | 20 April 2016 |
| | | | | WO | 2010-094212 | A2 | 26 August 2010 |
| | | | | WO | 2010-094212 | A3 | 06 January 2011 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210191745